# EUROPEAN PATENT APPLICATION

(11) **EP 2 514 413 A1**
(43) Date of publication of application: **24.10.2012**
(21) Application number: 12164996.6
(22) Date of filing: 20.04.2012
(51) Int. Cl.: A61K 9/20, A61K 31/64

(54) **Controlled-Release Gliclazide Formulations**

(30) Priority: 22.04.2011 TR 201103946
(71) Applicant: Sanovel Ilaç Sanayi Ve Ticaret Anonim Sirketi, 34460 Sariyer/Istanbul (TR)
(72) Inventor: Cifter, Ümit, 34460 Istanbul (TR); Türkyilmaz, Ali, 34460 Istanbul (TR); Pehlivan Akalin, Nur, 34460 Istanbul (TR); Sucuoglu, Esra, 34460 Istanbul (TR)
(74) Representative: Sevinç, Erkan

(57) **Abstract**

The present invention relates to a controlled-release tablet formulation comprising a therapeutically-effective amount of gliclazide or a pharmaceutically-acceptable salt thereof and at least one pharmaceutically-acceptable cellulosic polymer as a release rate-controlling polymer, characterized in that this tablet comprises cellulosic polymer at a proportion of 0.05 to 5% of the total weight of the tablet in its internal phase and at a proportion of 10 to 25% of the total weight of the tablet in its external phase.

## Description

### Field of Invention

The present invention relates to a controlled-release tablet formulation and to a method of producing the same. The present invention particularly relates to improving the stability of controlled-release formulations which comprise gliclazide.

### Background of Invention

Diabetes mellitus is an anomaly in glucose metabolism occurring due to causes like insufficient insulin secretion and decreased sensitivity of target cells to insulin and is characterized by hyperglycemia. In persistent hyperglycemia, severe harmful complications such as retinopathy, nephropathy, and neuropathy occur as a result of vascular lesions in various organs and nerves.

Many anti-diabetic medicaments, including sulfonylureas, have been administered orally in the treatment of diabetics so far.

Sulfonylurea is a name defining a group of orally administered anti-diabetic medicaments used in the treatment of type II diabetes mellitus. Diabetes mellitus is a disorder of the carbohydrate metabolism in which insulin secretion is diminished or which occurs due to a varying insulation secretion or decreased insulin activity or a combination of both factors. It is believed that sulfonylureas stimulate the insulin secretion from pancreatic islet cells in the mediation of receptors reported to be adenosine 5'-triphosphate-sensitive potassium channels. For this reason, sulfonylureas basically increase the endogenous insulin secretion so that an efficient control is provided in the blood sugar levels, not controllable via diets, of the diabetics and particularly of the type II diabetics.

Sulfonylureas are considered to be divided into two categories: first generation agents, e.g. tolbutamide, chlorpropamide, tolazamide, acetohexamide; and second generation agents, e.g. glyburide (glibenclamide), glipizide, gliclazide.

Gliclazide N-(4-methylbenzenesulfonyl)-N'-(3-azabicyclo[3.3.0]-oct-3-yl)urea), one type of second generation sulfonylureas, is an active agent which has antidiabetic properties at typical doses administered to humans (its chemical structure is illustrated with Formula 1). 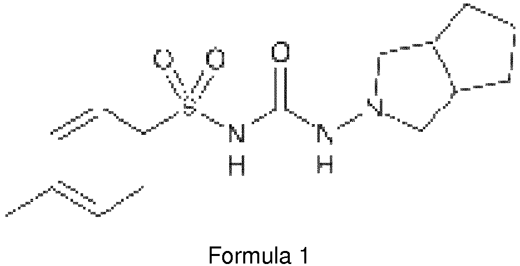 Gliclazide has been administered in the form of 80 mg conventional tablets until today. It is usually administrated one tablet twice a day making a total of two tablets. It may be altered, however, to 1 to 4 tablets a day, in line with the severity of the diabetes case. A 30 mg modified-release dosage form is also available at the markets.

The formulation of gliclazide was first disclosed on 10.12.1996 with the patent document US 3,501,495 (SCIENCE UNION ET CIE. SOC.), disclosing the formula N-(4-methylbenzenesulphonyl)-N'-(3-azabicyclo[3.3.0]-oct-3-yl)urea) with hypoglycemic properties, orally used in the treatment of diabetes mellitus.

Immediate-release formulations of sulfonylurea have been disclosed in many different patents in the past.

The patent US 4,696,815 discloses immediate-release tablets comprising sulfonylurea, formulated with an acidified and/or alkalized excipient and an inert polar solvent like polyethylene glycol. An analogous immediate-release formulation comprising an acidified and/or alkalized excipient, an inert polar solvent, and polyvinylpyrrolidone is also described in that patent.

The patent US 6,733,782 discloses a core tablet for the controlled-release of gliclazide, ensuring a sustained and constant release of the active agent by making use of hydroxypropylmethyl cellulose (HPMC) which is not influenced from the pH variations of the solubilizing medium following an oral administration. The main target of that invention is to obtain an oral dosage form, which can be administered once a day and provides extended release. That US patent provides information on using a glucose syrup, e.g. maltodextrin, together with cellulose polymer in order to obtain a controlled and full release from a gliclazide formulation. The use of an excipient like glucose syrup with a high glycemic index is typically not preferred in preparing a formulation for the treatment of diabetes mellitus.

In the patent US 6,703,045 is disclosed an oral controlled-release system, which is beneficial in lowering serum glucose levels. That patent also describes a method of lowering serum glucose levels with an oral controlled-release dosage form comprising glipizide and that a once-a-day dosage form ensures to keep the medicament at a therapeutic level throughout the day.

The patent EP 1 741 435 A1 relates to an oral modified-release tablet composition comprising a pharmaceutically effective amount of a micronized active ingredient for lowering blood glucose level and a hydrophilic polymer, as well as to a method of preparing a novel composition for that modified-release oral tablet.

With the application WO 2006/061697 A1 is disclosed sustained-release pharmaceutical formulations suitable for once-a-day administration, as well as a process for preparing such formulations comprising sulfonylurea, polymer, disaccharide and/or monosaccharide (lactose, sucrose, maltose, galactose, trehalose, maltitol, dextrose or the mixtures thereof) exhibiting a drug release profile substantially independent of pH of the dissolution medium in the pH range of 4 to 8.

The application WO 2006/123213 A1 relates to a modified-release formulation of gliclazide or a salt thereof and to a process for preparing the same. That pharmaceutical formulation comprises gliclazide or a salt thereof and the particle size of gliclazide may have a D90 range between less than about 70 µm to greater than about 18 µm or it may have a D90 less than about 18 µm. The formulation may comprise one or more controlled-release polymer, one or more binder, and optionally, one or more additional pharmaceutically-acceptable excipient. The formulations provide an extended and substantially controlled release of gliclazide or a salt thereof for a specific time interval.

The gliclazide active agent is quite susceptible to humidity. Particularly if they are formulated with polymers having a hydrophilic structure, extreme stability problems are encountered. While it is desired to obtain a desired release profile with hydrophilic polymers, the affinity of this polymer to humidity brings about stability-related problems. This fact is unfavorable in terms of pharmaceutics. Therefore, a formulation structure is needed that would overcome this problem.

### Description of Invention

The present invention provides a gliclazide formulation, eliminating all aforesaid problems and bringing additional advantages to the relevant prior art.

Accordingly, the main object of the present invention is to obtain a formulation of gliclazide, which is stable and has desired levels of solubility and dissolution rate.

Another object of the present invention is to provide a stable formulation of humidity-susceptible gliclazide molecule, having a structure that is not effected by humidity.

A further object of the present invention is to obtain a stable controlled-release formulation with high bioavailability.

In order to achieve the aforesaid objects and those objects to be disclosed in the following detailed description, a controlled-release tablet formulation is developed, which comprises a therapeutically-effective amount of gliclazide or a pharmaceutically-acceptable salt thereof and at least one pharmaceutically-acceptable cellulosic polymer as the release rate-controlling polymer.

In a preferred embodiment of the present invention, said novelty is realized in that said tablet comprises a cellulosic polymer in an amount corresponding to 0.5 to 5% of the total weight of the tablet in the internal phase and in an amount corresponding to 10 to 25% of the total weight of the tablet in the external phase.

In a preferred embodiment according to the present invention, the polymer that controls the release rate is selected from a group consisting of hydroxypropylmethyl cellulose (HPMC), hydroxypropyl cellulose (HPC), hydroxyethyl cellulose (HEC), ethyl cellulose (EC), methyl cellulose (MC), sodium carboxy methyl cellulose, and cellulose acetate butyrate, or a mixture thereof.

In another preferred embodiment according to the present invention, the polymer that controls the release rate is hydroxypropylmethyl cellulose.

In a preferred embodiment according to the present invention, the viscosity of the hydroxypropylmethyl cellulose polymer that controls the release rate is 100 cP.

In another preferred embodiment according to the present invention, the viscosity of the hydroxypropylmethyl cellulose polymer that controls the release rate is 4000 cP.

In a preferred embodiment according to the present invention, the viscosity of the hydroxypropylmethyl cellulose present in the internal phase is 4000 cP.

In a preferred embodiment according to the present invention, the weight proportion of hydroxypropylmethyl cellulose in the internal phase to hydroxypropylmethyl cellulose in the external phase is in the range of 0.02 to 0.5.

In another preferred embodiment according to the present invention, the total amount of the polymer that controls the release rate amounts to 10 to 30% of the tablet weight.

Another preferred embodiment according to the present invention further comprises at least one or a mixture of fillers, glidants, and lubricants as an excipient.

In another preferred embodiment according to the present invention, said filler is at least one or a mixture of calcium hydrogen phosphate dihydrate, calcium hydrogen phosphate anhydrate or mannitol.

In another preferred embodiment according to the present invention, said glidant is colloidal silicon dioxide.

In another preferred embodiment according to the present invention, said lubricant is magnesium stearate.

In another preferred embodiment according to the present invention, said formulation does not contain glucose syrup.

According to another preferred embodiment of to the present invention, said formulation consisting of
**i- internal phase**
   a. gliclazide or a pharmaceutically-acceptable salt or polymorph thereof at 15 to 22% by weight,
   b. hydroxypropylmethyl cellulose with a viscosity of 4000 cP at 1.8 to 3.0% by weight,
   c. calcium hydrogen phosphate dihydrate at 12 to 25% by weight,
   d. mannitol at 35 to 55% by weight,
**ii- external phase**
   a. hydroxypropylmethyl cellulose with a viscosity of 4000 cP at 7.5 to 12% by weight,
   b. hydroxypropylmethyl cellulose with a viscosity of 100 cP at 7.5 to 9.0% by weight,
   c. colloidal silicone dioxide at 0.05 to 0.5% by weight,
   d. magnesium stearate at 0.25 to 1.0% by weight.

### Example

A controlled-release pharmaceutical tablet formulation of gliclazide

This example describes a controlled-release pharmaceutical tablet formulation comprising gliclazide or a pharmaceutically-acceptable salt thereof. The active ingredient and excipients of the tablet are given below:
Controlled-release tablet formulation

| | amount (%) |
|---|---|
| gliclazide | 15-22 |
| hydroxypropylmethyl cellulose 4000 cP | 1.8 - 3.0 |
| calcium hydrogen phosphate dihydrate | 12-25 |
| hydroxypropylmethyl cellulose 100 cP | 7.5 - 9.0 |
| mannitol | 35 - 55 |
| colloidal silicone dioxide | 0.05 - 0.5 |
| magnesium stearate | 0.25 - 1.0 |

Gliclazide, calcium hydrogen phosphate dihydrate, mannitol, and some portion of hydroxypropylmethyl cellulose (4000 cP) (E4M) are mixed. Water is sprayed onto the resulting mixture and is thus granulated.

The wet granules prepared are directly transferred to a fluidized bed dryer. The granules are dried in the fluidized bed dryer until the humidity of granules are reduced to below 1% maximum, and dried granules are sieved and passed to the mixer.

The remaining part of hydroxypropylmethyl cellulose (E4M), the entirety of hydroxypropylmethyl cellulose (K100LV), and colloidal silicone dioxide are added to the sieved dry granules and the resulting mixture is mixed. Then, magnesium stearate is added therein and mixing is continued. The mixture is compressed into tablets.

Thus, a controlled-release formulation is obtained with a surprisingly improved stability and desired levels of solubility and dissolution rate. Thanks to this tablet embodiment in which hydroxypropylmethyl cellulose (4000 cP) is used at different amounts in the internal phase and external phase, the stability of the formulation is surprisingly maintained and no differences are observed in the dissolution profiles of the formulation. The 100 cP hydroxypropylmethyl cellulose is used in the external phase. The weight proportion of the 4000 cP viscosity hydroxypropylmethyl cellulose to the 100 cP viscosity hydroxypropylmethyl cellulose is in the range of 0.02 to 0.5 and this proportion is critical in terms of providing a balance between the stability and the release profile of the formulation.

The poise (symbol P) is the unit of dynamic viscosity in the centimetre gram second system of units. Centipoise is properly abbreviated cP, but the alternative abbreviations cps, cp, and cPs are also commonly seen. Water has a viscosity of 0.00899 Poise at 25 °C and a pressure of 1 atmosphere. (0.00899 P = 0.899cP = 0.899 mPa·s)

This formulation is used for preventing or treating diabetes mellitus in mammalians, and particularly in humans.

It is also possible to use the terms "modified-release", "extended-release", "prolonged-release", and "sustained-release" in place of the term "controlled-release".

The polymers can be used to form a matrix in which the active agent is dispersed.

The release rate of the active agent depends on the properties of the polymer used. The cellulosic derivates are particularly hydroxypropylmethyl cellulose (HPMC) and hydroxypropyl cellulose (HPC), see "Handbook of Pharmaceutical Excipients (Ed. A Wade and P. J. Weller, Pharmaceutical Press, London, 1994). The properties of a polymer when it contacts an aqueous medium and particularly a physiological medium can be used for controlling the release rate of an active agent from a controlled-release formulation. For instance, polymers like HPMC frequently develop a gel-like outer layer that is dissolved or worn away and thus control the permeation of water into the interior of a controlled-release formulation. It is known that the release of an active agent from such controlled-release formulations is controlled by a combination of many factors, including the properties of the active agent, the diffusion properties of the active agent or of the solution in which it is just dissolved, the penetration rate of water into the interior of the polymer matrix, swelling features of the polymer matrix, and the dissolution rate of the gel layer of the outer polymer.

The present invention makes use of mannitol as a binder and a hydrophilic diluent with a low glycemic index.

The preferred embodiment of this invention is a controlled-release pharmaceutical tablet formulation, comprising (a) a therapeutically-effective amount of gliclazide or a pharmaceutically-acceptable salt thereof, (b) mannitol as a binder and hydrophilic diluent, not increasing the blood glucose level in human patients who are in the need of a non-insulin dependent diabetes mellitus treatment (NIDDM), (c) calcium hydrogen phosphate dihydrate or calcium hydrogen phosphate anhydrate as a diluent, (d) two different types of pharmaceutically-acceptable cellulosic polymers as a release rate-controlling polymer, wherein not more than 25% of the total amount of gliclazide or a pharmaceutically-acceptable salt thereof is dissolved in two hours and wherein the blood glucose level is not increased.

The pharmaceutically-acceptable cellulosic polymer controlling the release rate may comprise HPMC, HPC, hydroxyethyl cellulose (HEC), ethyl cellulose (EC), methyl cellulose (MC), sodium carboxymethyl cellulose and cellulose acetate butyrate or a mixture thereof.

The preferred polymer according to the present invention is HPMC, used as a release rate-controlling polymer. According to a most preferred embodiment, the viscosity of HPMC is 100 cP and 4000 cP.

The amount of the release rate-controlling polymer is 10 to 30%, the amount of calcium hydrogen phosphate dihydrate or calcium hydrogen phosphate anhydrate is 12 to 25% and the amount of mannitol is 35 to 55% in the controlled-release pharmaceutical tablet formulation according to the present invention.

According to a preferred embodiment of this invention, the controlled-release pharmaceutical tablet formulation further comprises at least one excipient. This excipient is selected from a group consisting of glidants and lubricants.

As mentioned above, said glidant may be selected from a group consisting of colloidal silicone dioxide, silica, calcium silicate, magnesium trisilicate, sodium stearyl fumarate, talk, etc.. The glidant is preferably colloidal silicone dioxide used in an amount of 0.05 to 0.5%.

As mentioned above, said lubricant is selected from a group consisting of magnesium stearate, calcium stearate, stearic acid, hydrogenated vegetable oils, vegetable-derived fatty acids, etc.. The lubricant is preferably magnesium stearate used in an amount of 0.25 to 1.0%

According to a most preferred embodiment of this invention, conducting an *in vitro* dissolution test by an USP pedal method in a 900 ml medium (0.05 M pH 7.5 phosphate buffer) at 75 rpm and 37°C should provide a dissolution rate for calcium hydrogen phosphate dihydrate or calcium hydrogen phosphate anhydrate and mannitol and HPMC and gliclazide or a pharmaceutically-acceptable salt thereof not more than 25% in 2 hours, 30 to 65% in 6 hours, and not less than %85 in 24 hours.

As mentioned above, the cellulosic polymer is hydroxypropylmethyl cellulose 100 cP or 4000 cP used in an amount of 10 to 30%, the amount of calcium hydrogen phosphate dihydrate or calcium hydrogen phosphate anhydrate is 12 to 25% and the amount of mannitol is 35 to 55%.

The problems related to the prior art are solved with the present invention and the objects referred to hereinabove of this invention relates to a novel controlled-release pharmaceutical tablet formulation.

This invention is hereby disclosed by referring to exemplary embodiments hereinabove. Whilst these exemplary embodiments does not restrict the object of the present invention, the latter must be assessed under the light of the foregoing detailed description.

## Claims

1. A controlled-release tablet formulation comprising a therapeutically-effective amount of gliclazide or a pharmaceutically-acceptable salt thereof and at least one pharmaceutically-acceptable cellulosic polymer as a release rate-controlling polymer, **characterized in that** this tablet comprises cellulosic polymer at a proportion of 0.05 to 5% of the total weight of the tablet in its internal phase and at a proportion of 10 to 25% of the total weight of the tablet in its external phase.

2. The controlled-release tablet formulation according to Claim 1, wherein the release rate-controlling polymer is selected from a group consisting of hydroxypropylmethyl cellulose (HPMC), hydroxypropyl cellulose (HPC), hydroxyethyl cellulose (HEC), ethyl cellulose (EC), methyl cellulose (MC), sodium carboxy methyl cellulose, and cellulose acetate butyrate, or a mixture thereof.

3. The controlled-release tablet formulation according to claims 1 and 2, wherein the release rate-controlling polymer is hydroxypropylmethyl cellulose.

4. The controlled-release tablet formulation according to claims 1, 2, and 3, wherein the viscosity of the hydroxypropylmethyl cellulose polymer controlling the release rate is 100 cP.

5. The controlled-release tablet formulation according to claims 1, 2, and 3, wherein the viscosity of the hydroxypropylmethyl cellulose polymer controlling the release rate is 4000 cP.

6. The controlled-release tablet formulation according to Claim 1, wherein the viscosity of the hydroxypropylmethyl cellulose present in the internal phase is 4000 cP.

7. The controlled-release tablet formulation according to Claim 1, wherein the proportion of hydroxypropylmethyl cellulose in the internal phase to hydroxypropylmethyl cellulose in the external phase is in the range of 0.02 to 0.5.

8. The controlled-release tablet formulation according to claims 1 and 2, wherein the amount of the release rate-controlling polymer is 10 to 30%.

9. The controlled-release tablet formulation according to Claim 1, further comprising at least one or a mixture of binders, glidants and lubricants as an excipient.

10. The controlled-release tablet formulation according to claims 1 and 8, wherein said filler is at least one or a mixture of calcium hydrogen phosphate dihydrate, calcium hydrogen phosphate anhydrate or mannitol.

11. The controlled-release tablet formulation according to claims 1 and 8, wherein said glidant is colloidal silicone dioxide.

12. The controlled-release tablet formulation according to claims 1 and 8, wherein said lubricant is magnesium stearate.

13. The controlled-release tablet formulation according to Claim 1, **characterized in that** this tablet formulation does not comprising any glucose syrup.

14. The controlled-release tablet formulation according to Claim 1, consist of
i. internal phase
a. gliclazide or a pharmaceutically-acceptable salt or polymorph thereof at 15 to 22% by weight,
b. hydroxypropylmethyl cellulose with a viscosity of 4000 cP at 1.8 to 3.0% by weight,
c. calcium hydrogen phosphate dihydrate at 12 to 25% by weight,
d. mannitol at 35 to 55% by weight,
ii. external phase
a. hydroxypropylmethyl cellulose with a viscosity of 4000 cP at 7.5 to 12% by weight,
b. hydroxypropylmethyl cellulose with a viscosity of 100 cP at 7.5 to 9.0% by weight,
c. colloidal silicone dioxide at 0.05 to 0.5% by weight,
d. magnesium stearate at 0.25 to 1.0% by weight.

15. The controlled-release tablet formulation according to Claim 1 for preventing or treating diabetes mellitus in mammalians and particularly in humans.
